# NEUE EUROPÄISCHE PATENTSCHRIFT

(11) **EP 0 925 060 B2**
(45) Veröffentlichungstag und Bekanntmachung des Hinweises auf die Entscheidung über den Einspruch: **16.06.2010**
(45) Hinweis auf die Patenterteilung: 04.12.2002
(21) Anmeldenummer: 97943848.8
(22) Anmeldetag: 09.09.1997
(51) Int. Cl.: A61K 9/22, A61K 9/52

(54) **SCHNELLZERFALLENDE PELLETS**
FAST DECOMPOSING PELLETS
PASTILLES SE DECOMPOSANT RAPIDEMENT

(30) Priorität: 12.09.1996 DE 19637082
(43) Veröffentlichungstag der Anmeldung: 30.06.1999
(73) Patentinhaber: Roche Diagnostics GmbH, 68305 Mannheim (DE)
(72) Erfinder: OPITZ, Michaela, D-67098 Bad Dürkheim (DE); VON BÜREN, Hendrik, D-69226 Nu loch (DE); GABEL, Rolf-Dieter, D-68723 Schwetzingen (DE); LEE, Geoffrey, D-91054 Buckenhof (DE)
(74) Vertreter: Best, Michael
(86) Internationale Anmeldenummer: PCT/EP1997/004897
(87) Internationale Veröffentlichungsnummer: WO 1998/010754

(56) Entgegenhaltungen:
- EP-A- 0 421 921
- WO-A-92/00064
- WO-A-95/28147
- DE-A- 3 532 692
- FARM. VESTN. Bd. 46, 1995, Seiten 211 - 212
- ACTA PHARM. SUEC. Bd. 18, 1981, Seiten 108 - 109
- EUR. J. PHARM. BIOPHARM. Bd. 41, Nr. 6, 1995, Seiten 382 - 387
- PHARM. SCIENCES 1995, Seiten 415 - 418
- INT. J. PHARMACEUTICS Bd. 96, 1993, Seiten 119 - 128
- DRUG. DEV. IND. PHAR. Bd. 19, Nr. 8, 1993, Seiten 915 - 927

## Beschreibung

Die Erfindung betrifft pharmazeutische Darreichungsformen in Form von Pellets, die mindestens ein als Retardierungs- oder Zerfallsverzögerungsmittel wirkendes, zur Herstellung von Extrusionspellets erforderliches Rundungsmittel enthalten, das geeignet ist, die zeitliche Freisetzung von Wirkstoffen zu verzogern, wobei die Freisetzung des Wirkstoffes aus dem Pellet (Pellet A) nicht geringer ist im Vergleich zu einem entsprechenden Referenzkernpellet (Pellet B), das dieses Rundungsmittel als pharmazeutischen Hilfsstoff nicht enthalt. Die Freisetzungsrate dieser schnellzerfallenden Pellets betragt mindestens etwa 90 % innerhalb einer Zeitspanne von 30 Minuten. Gegenstand der vorliegenden Erfindung sind ferner Verfahren zur Herstellung dieser Pellets.

Pellets werden zumeist für modifiziert freisetzende Arzneiformen eingesetzt. Sie besitzen im Vergleich zu herkömmlichen Arzneiformen mit modifizierter Wirkstofffreisetzung deutliche Vorteile, wie z.B. die Vermeidung des dose-dumping und lokaler Unverträglichkeiten, Minimierung intra- und interindividueller Schwankungen, Unabhängigkeit von Magenentleerungszeiten, Mischung verschieden retardierter Pellets. Mischung von Pellets mit verschiedenen (ggf. inkompatiblen) Wirkstoffen und Verbesserung der Bioverfügbarkeit.

Folgende Prinzipien werden zur Herstellung von Pellets mit modifizierter Wirkstofffreisetzung häufig eingesetzt: a) Modifizierung der Wirkstoffabgabe mit Hilfe von Überzügen oder b) Matrixsysteme.

Magensaftresistente Umhüllungen sind im sauren Milieu des Magens stabil und lösen sich durch Salzbildung im schwach sauren bzw. basischen Bereich langsam auf. Da Pellets jedoch von den Magenentleerungsrhythmen weitgehend unabhängig sind, fuhren magensaftresistente Überzuge nur zu einer kurzfristigen Verzögerung der Wirkstofffreisetzung.

Des weiteren werden Überzuge eingesetzt, die im Magen-Darm-Trakt unlöslich sind und den gelosten Wirkstoff mittels Diffusion durch die Hülle freisetzen. Hierbei läßt sich die Freisetzungsgeschwindigkeit z.B. über den Diffusionskoeffizienten, die Filmdicke, das Konzentrationsgefälle, den osmotischen Druck und die Verwendung von Porenbildnern einstellen. Bei schwerlöslichen Wirkstoffen reicht jedoch die durch die Hülle in den Kern diffundierende Flüssigkeitsmenge nicht zur Auflösung des Wirkstoffes aus, so daß nur ein Teil der Gesamtdosis freigesetzt wird.

Um diese Nachteile zu umgehen, wurden in den letzten Jahren zeitgesteuerte Systeme entwickelt, bei denen die Umhüllung nach einer bestimmten Verzögerungszeit aufplatzt. Durch das Mischen verschieden umhüllter Pellet-Kollektive, läßt sich das Freisetzungsprofil einstellen. Diese Systeme zeichnen sich durch ihre Unabhängigkeit von intra- und interindividuellen Schwankungen aus, setzen die komplette Wirkstoffdosis frei und eignen sich sowohl für leichtlosliche als auch schwerlosliche Arzneistoffe.

Milosovich (US 3,247,066) entwickelte eine Arzneiform mit kontrollierter Wirkstofffreigabe auf der Basis kleiner Kügelchen, die ein in Wasser quellendes Kolloid enthalten und mit einer unverdaulichen Hülle überzogen sind. Mittels Diffusion gelangt Verdauungsflüssigkeit in den sprengmittelhaltigen Kern, welcher daraufhin quillt und den Kern zum Platzen bringt.

Eine weitere Variante ist das mehrschichtig aufgebaute sogenannte "Time Controlled Explosion System" (abgekürzt: TCES) (vgl. EP 0 210 540 B1), bei dem eine sprengmittelhaltige Schicht direkt unter der Umhüllung das Platzen des Überzuges veranlaßt.

Die Bayer AG (Patent DD 297 767) entwickelte eine durch Rotorgranulation hergestellte Pelletformulierung mit zeitlich gesteuerter Freigabe. Mit Hilfe einer freisetzungskontrollierenden Doppelschicht aus einer äußeren unverdaulichen Lackschicht und einem inneren Mantel, der die Migration des Wassers in Richtung Kern kontrolliert, gelangt Feuchtigkeit in den sprengmittelhaltigen Kern, der daraufhin die Umhüllung sprengt.

Die in US 3,247,066, in EP 0 210 540 B1 und DD 297 767 hergestellten Formulierungen wurden allerdings nicht mittels Extrusion und Rundung hergestellt, so daß keine Pellets mit enger Korngrößenverteilung erhalten werden können.

Aus EP 0 421 921 B1 sind doppelt beschichtete Granulate bekannt, die man durch Extrusion der feuchten Granuliermasse erhält und die als sphärische Pellets mit einem Durchmesser von 0.3 bis 1,5 mm geformt sind. Allerdings handelt es sich dabei um Pellets, deren zeitliche Steuerung der Freisetzung durch einen magensaftresistenten, aber darmsaftlöslichen Film erreicht wird. und nicht durch einen "burst-Mechanismus".

In der Druckschrifft "Pharmaceutical Sciences", 1995, 1:415-418 wird das Problem angesprochen, bei Extrusionspellets eine schnelle Freisetzung und einen schneller Zerfall zu erhalten. Hierzu wird vorgeschlagen, Pellets mit dem Sprengmittel Natrium stärke glykolat in einer 2-Propanal-haltigen Granulatsausflüssigkeit herzustellen.

Obwohl Extrusionsverfahren gegenuber der Rotorgranulation den Vorteil haben, daß enge Korngrößenverteilungen erreichbar sind, besteht ein Nachteil von Extrusionspellets grundsätzlich darin, daß bei ihrer Herstellung auf bestimmte pharmazeutische Zusatzstoffe, wie zum Beispiel mikrokristalline Cellulose, als Rundungshilfsmittel nicht verzichtet werden kann, da sie dem Extrudat die zur Rundung erforderlichen plastischrigiden Eigenschaften verleiht. Derartige Zusatzstoffe wirken dabei in vielen Fällen auch als Retardierungsmittel, d.h. sie führen zu einer zeitlich verzögerten (retardierten) Freisetzung des Wirkstoffes. Diese oft zwangsläufig erfolgende Retardierung ist nicht in allen Fällen erwünscht. Retardierungsmittel, insbesondere mikrokristalline Cellulose, können ein Matrixsystem ausbilden, das den Zerfall der Pellets verhindert, so daß insgesamt Pellets mit retardierenden Eigenschaften bezüglich der Wirkstofffreisetzung erhalten werden. Die als Rundungshilfsmittel verwendeten Zusatzstoffe können ferner als Zerfallsverzögerungsmittel wirken, d.h. sie verhindern den raschen Zerfall der Pellets in kleinere Partikel. Diese Effekte bewirken vor allem bei schwerlöslichen Arzneistoffen eine starke zeitliche Verzögerung in der Wirkstofffreisetzung und eine Retardierung der Arzneistoffabgabe. So sind Wirkstoffe, die ein stark pH-abhängiges Löslichkeitsprofil zeigen und besonders im basischen Darmsaft schlecht löslich sind, durch die Ausbildung eines dichten Matrixsystems mit mikrokristalliner Cellulose gekennzeichnet. In solchen Fällen läßt sich das Freigabeprofil nicht beliebig über die Zusammensetzung und die Dicke des Überzuges variieren, da das Herauslosen des Arzneistoffes aus der Matrix bzw. die Geschwindigkeit des Zerfalls der Pellets entscheidend zum Freisetzungsverhalten beitragen.

Der Erfindung lag deshalb die Aufgabe zugrunde, solche Pellets zur Verfügung zu stellen, die schnell zerfallen und den Wirkstoff in moglichst kurzer Zeit aus den Kernpellets freisetzen, obwohl die Pellets Rundungshilfsmittel beinhalten, die unter anderem auch als Retardierungsmittel und/oder Zerfallsverzögerungsmittel fungieren.

Überraschend wurde festgestellt, daß Pelletkerne, die a) ein als Retardierungsmittel oder Zerfallsverzögerungsmittel wirkendes Rundungsmittel, b) ein Tablettensprengmittel (im folgenden auch als Intensivsprengmittel bezeichnet) und c) mindestens einen Hilfsstoff ausgewählt aus der Gruppe bestehend aus Tensiden und Bindemitteln, wobei das Bindemittel Polyvinylpyrrolidon ist sowie d) gegebenenfalls Füllmittel oder Kombinationen dieser Hilfsstoffe, gut zerfallen. Die entsprechenden Wirkstoffe werden außerdem aus den Kernpellets schnell und im wesentlichen ohne zeitlich verzögerte Freisetzung im Vergleich zu einem Pellet, das dieses Rundungsmittel bzw. Retardierungsmittel nicht besitzt, freigesetzt. Dies trifft insbesondere zu für schwer lösliche Wirkstoffe. Insbesondere enthalten die Kernpellets neben dem Rundungsmittel ein Intensivsprengmittel, ein Tensid und ein Bindemittel. Anstelle des Tensides kann auch Polyvinylpyrrolidon (PVP), eingesetzt werden. In einer bevorzugten Variante wird ein PVP zusatzlich neben dem Tensid dem Pelletkern zugefügt.

Die erfindungsgemäßen Pellets haben ferner den Vorteil, daß sie eine enge Korngrößenverteilung aufweisen: Mindestens 90 % der Teilchen besitzen dabei einen Durchmesser von etwa 0,6 - 1,2 mm. Darüberhinaus zerfallen die Pellets auch mit solchen Wirkstoffen relativ schnell, die ein stark pH-abhangiges Löslichkeitsprofil zeigen. Die Pellets besitzen üblicherweise einen Durchmesser zwischen 0,5 - 2 mm, je nach Größe der bei der Extrusion verwendeten Lochscheibe.

Die erfindungsgemäßen schnellzerfallenden Pellets (im folgenden auch als Kernpellet A bezeichnet) weisen eine Freisetzungsrate des Wirkstoffes auf, die trotz des Vorhandenseins des als Retardierungs- oder Zerfallsverzögerungsmittel wirkenden Rundungsmittel nicht verzögert ist. Die Freisetzung des Wirkstoffes ist dabei im wesentlichen nicht retardiert. Die Freisetzung erfolgt relativ schnell und ist insbesondere vergleichbar mit einem Pellet, das auch nach einem anderen Verfahren als nach dem Extrusionsverfahren hergestellt werden kann, und das dieses Retardierungs- oder Zerfallverzogerungsmittel nicht enthält (im folgenden auch als Kernpellet B oder Referenzpellet bezeichnet). Die erfindungsgemäßen Kernpellets weisen keine verzögerte Freisetzung des Wirkstoffes auf. Die Freisetzungsrate derartiger Kernpellets beträgt vorzugsweise mindestens 90 % nach 30 Minuten.

Die erfindungsgemäßen Pellets (Kernpellets A) enthalten die pharmazeutisch üblichen Sprengmittel vorteilhaft in einer Menge von 5 - 50 % und die Tenside in einer Menge von 0,1 - 20%. Bindemittel können vorteilhaft in einer Menge von 1 - 10 % zugesetzt werden. Mikrokristalline Cellulose (z.B. Avicel®) als Rundungshilfsmittel ist insbesondere von 5 - 70 % vorhanden. Die Prozentangaben beziehen sich auf Gewichtsprozent der Pelletkerne, sofern nichts anderes angegeben ist.

Als Rundungshilfsmittel für die Herstellung von Extrusionspellets eignen sich alle üblichen, in der Literatur bekannten Hilfsstoffe, die ein Abrunden der durch die Extrusion primär in Stäbchenförm erhaltenen Arzneimittelmassen ermoglichen. Beispielhaft kommt mikrokristalline Cellulose und deren Derivate, wie z.B. Avicel®, Avicel® PH 101, Avicel® PH 105 oder Avicel® PH 200, in Frage. Sofern diese Rundungshilfsmittel eine zeitlich verzogerte Freisetzung des Wirkstoffes im Vergleich zu anderen Pellets bewirken, die diesen Hilfsstoff nicht enthalten, oder diese Rundungshilfsmittel den Zerfall der Pellets in kleinere Partikel verzögern (Zerfallsverzögerungsmittel), kommen sie im Sinne der vorliegenden Erfindung als sog. "Retardierungsmittel" zur Herstellung der erfindungsgemäßen Kernpellets in Frage.

Als Bindemittel ist Polyvinylpyrrolidon (PVP) vorhenden. Polyvinylpyrrolidon (PVP) beeinträcht ist den Zerfall der Pellets trotz hervorragender Bindeeigenschaften nicht, sondern begunstigt ist, so daß erfindungsgemäß beim Einsatz von PVP in Kombination mit einem Intensivsprengmittel sogar auf das Tensid verzichtet werden kann.

Als Tablettensprengmittel (Zerfallshilfsmittel) bzw. Intensivsprengmittel für pharmazeutische Zwecke können alle pharmazeutisch üblichen Hilfsmittel eingesetzt werden, die in wässrigen Medien eine stark quellende Eigenschaft besitzen und sich durch eine Volumenvergrößerung bei Aufnahme von Wasser auszeichnen. Tablettensprengmittel ist eine Bezeichnung für solche pharmazeutische Hilfsstoffe, die für den raschen Zerfall von Tabletten in Wasser oder im Magensaft und für die Freisetzung der Pharmaka in resorbierbarer Form sorgen. Je nach Wirkungsmechanismus handelt es sich um Substanzen, die die Porosität der festen Darreichungsformen erhöhen und ein großes Adsorptionsvermögen fiir Wasser besitzen (Stärke, Cellulose-Deriv., Alginate, Dextrane, quervernetztes Polyvinylpyrrolidon u.a.), sowie Hydrophilierungsmittel, die für die Benetzung der festen Darreichungsformen sorgen (wie z.B. Polyethylenglykolsorbitanfettsäureester). Bevorzugt wird Natrium-Carboxymethylcellulose, modifizierte Maisstärke (z. B. Starch® 1500), und Na-Carboxymethylstärke (Explotab® bzw. Primojel®) eingesetzt. Besonders bevorzugt ist Primojel®.

Als Tenside werden pharmazeutisch übliche grenzflächenaktive Stoffe eingesetzt, so ionische und nicht ionische Tenside, wie Benzalkoniumchlorid, Polyoxyethylen-Polyoxypropylen-Copolymere, (z.B.Pluronic® F 68), Polyethylenglykol-glyceryl-ester, Alkylsulfate, vorzugsweise Na-Dodecylsulfat (Texapon®), und Stearinsäure oder deren Alkali- oder Erdalkalisalze (Mg- oder Na-salze) bzw. Stearate wie z.B. PEG- 400-Stearat (Mirj®) Gegebenenfalls werden außerdem ein oder mehrere phamazeutisch übliche Füllmittel zugegeben. Der Füllmittelanteil kann bis zu 80% betragen. Als Füllmittel werden erfindungsgemäß Kohlenhydrate, wie Zucker, vorzugsweise Glucose, Lactose und Saccharose, Zuckeralkohole, wie Mannit und Sorbit, Stärke, Stärkederivate und Dibasisches Calciumphosphat eingesetzt. Prinzipiell sind jedoch alle bekannten Füllmittel geeignet.

Erfindungsgemäß besonders bevorzugte Formulierungen umfassen 15 - 25 % mikrokristalline Cellulose, 15 - 25 % Sprengmittel, 2 - 10 % Tensid und/oder 3 - 7% Polyvinylpyrrolidon (Angaben jeweils in Gew.-%) sowie gegebenenfalls weitere Binde- oder Füllmittel.

Wirkstoffe im Sinne der Erfindung sind grundsätzlich alle Arzneistoffe, die für die therapeutische Behandlung von Menschen in Frage kommen. Vorteilhaft eignen sich solche Wirkstoffe, die schwer löslich sind. Schwer lösliche Wirkstoffe im Sinne der vorliegenden Erfindung sind solche, die in den allgemeinen Arzneimittelbüchern (z.B. USP XXII) als nicht leicht lösliche Wirkstoffe bezeichnet werden. Derartige Wirkstoffe weisen beispielsweise eine Löslichkeit von weniger als 0,1 mg/ml, insbesondere weniger als 0,05 mg/ml oder weniger als 0,01 mg/ml im wässrigem Medium auf oder besitzen eine starke pH-Abhängigkeit im Löslichkeitsverhalten. Beispielhafte Wirkstoffe sind (+/-)-1-(9H-Carbazol-4-yloxy)-3-[(2-(2-methoxyphenoxy)-ethyl)-amino]-2-propanol (INN: Carvedilol), 2-{4-[2-[(4-chloro-benzoyl)-amino]ethyl]-phenoxy}-2-methyl-propionsaure (INN: Bezafibrat), INN: Glibenclamid oder 1-Isopropyl-3-[(4-m-toluidino-3-pyridyl)sulfonyl]-urea (INN. Torasemid). Diese Wirkstoffe sind schwer löslich. Insbesondere Carvedilol zeigt ein stark pH-abhängiges Löslichkeitsprofil und ist besonders im Darmsaft schlecht löslich. Aus den erfindungsgemäßen Pellets werden diese Wirkstoffe, und insbesondere Carvedilol, jedoch sehr gut freigesetzt.

Zur Prüfung des Zerfalls von Pellets können allgemein anerkannte Verfahren bzw. Apparaturen verwendet werden, die in Arzneimittelbüchern in standardisierter Form beschrieben sind. Zur Messung der Zerfallszeit kann auch die entsprechend standardisierte Paddle-Apparatur (37 °C, 90 Upm) eingesetzt werden. Sobald mindestens 90% der Pellets in kleinere Agglomerate zerfallen sind, wird die Zerfallszeit abgelesen. Die Freisetzungsrate des Wirkstoffes (Angabe in % bezogen auf eine bestimmte Zeiteinheit) wird ebenfalls nach allgemein standardisierten Verfahren (vgl. European Pharmacopoea oder US Pharmacopoea) bestimmt.

Die erfindungsgemäßen Pellets weisen überraschend eine Zerfallsrate von mindestens 90 % nach 30 Minuten auf, in besonders bevorzugten Ausführungsformen bereits nach 20, 10 oder 5 oder 2 Minuten. Die Wirkstofffreisetzung erfolgt zu mindestens 90 % nach 30 Minuten. Nach 10 Minuten beträgt die Freisetzung des Wirkstoffes aus den Kernpellets mindestens 50 %, bevorzugt mindestens 70 % und insbesondere mindestens 90 %. Diese Freisetzungsraten können überraschenderweise auch für schwer lösliche Wirkstoffe erzielt werden. Die Freisetzung wird in wässrigem Medium bestimmt, wobei der pH-Wert der Lösung auf einen Wert eingestellt wird, bei dem der Wirkstoff eine optimale Löslichkeit besitzt.

So zerfallen Texapon®- und sprengmittelhaltige Carvedilol-Pellets bereits sogar schon nach 2 Minuten und zeigen eine Wirkstofffreisetzung von ca. 70 % bereits nach 5 Minuten. Durch Anwendung von Pluronic® F 68 wird günstigerweise der Zerfall noch beschleunigt, wobei bereits nach 5 Minuten mehr als 90 % des Carvedilols freigesetzt werden. Diese erfindungsgemäßen, nicht überzogenen Pelletkerne eignen sich somit als Alternative zu einer nicht retardierten monolithischen Arzneiform.

Die erfindungsgemäßen Pellets enthalte eine Kombination eines Tablettensprengmittels zusammen mit entweder einem Tensid und/oder den Bindenmittel PVP.

Die erfindungsgemäßen Pellets werden hergestellt, indem man die Wirkstoffe mit den pharmazeutischen Hilfsstoffen 1 mischt, anschließend granuliert, extrudiert und rundet. Das erfindungsgemäße Extrusions/Rundungsverfahren ermöglicht im Gegensatz zur Rotorgranulation die Herstellung von Pellets mit sehr enger Korngrößenverteilung. Bei Verwendung einer Lochscheibe mit einem Lochdurchmesser von 1 mm weisen etwa 90 % der Pellets einen Durchmesser zwischen 0,6 - 1,2 mm auf.

Da bei der Herstellung der erfindungsgemäßen Pellets kein neutraler Starterkern erforderlich ist, lassen sich viel größere Wirkstoffdosen einarbeiten. Gemäß der Erfindung sind Wirkstoffmengen bis zu 80 % (Gew.-%) realisierbar. Vorzugsweise betragt der Wirkstoffanteil beispielsweise mindestens 30 %, 50 % oder 70 %. Carvedilol-Pellets mit einem Wirkstoffanteil von 70 % können im Sinne der vorliegenden Erfindung völlig problemlos hergestellt werden und zerfallen innerhalb von weniger als 10 Minuten, insbesondere weniger als 5 Minuten bzw. weniger als 2 Minuten.

In einer besonderen Ausführungsform können die erfindungsgemäßen Kernpellets auch mit Umhüllungen überzogen werden, z. B. um die Wirkstofffreisetzung zu modifizieren oder einen unangenehmen Geschmack zu kaschieren. Im Sinne der vorliegenden Erfindung können die schnell zerfallenden Kernpellets auch mit einer Umhüllung versehen werden, um zeitgesteuerte Systeme zu entwickeln, bei denen die Umhüllung nach einer bestimmten Verzögerungszeit aufplatzt. Die erfindungsgemäßen Kernformulierungen sind besonders gut geeignet als Grundlage für die Entwicklung einer Arzneiform mit modifizierter Wirkstofffreigabe, bei der durch das Aufplatzen eines Filmes eine zeitlich kontrollierte Wirkstofffreigabe erreicht wird, da der im Pelletkern nach Feuchtigkeitsaufnahme entwickelte Quellungsdruck ausreicht, um einen unverdaulichen Überzug aufzureißen. Derartige "burst-Systeme" bieten insbesondere bei schwer wasserlöslichen Arzneistoffen den Vorteil gegenüber Diffusionspellets, daß die Wirkstoffdosis aus der Arzneiform vergleichsweise rasch und vollständig freigesetzt wird. Durch die Zusammensetzung und die Filmauftragsdicke des Filmes sowie durch die Rezeptur des schnellzerfallenden Kerns, läßt sich die Verzogerungszeit variabel zwischen 10 Minuten und 5 Stunden einstellen.

Es ist bekannt, daß eine dicke Filmschicht alleine zwar den Start der Freisetzung lange verzögern kann, dadurch aber nach dem Platzen der Hülle eine langsamere Wirkstofffreisetzung resultiert. Hohe Auftragsmengen erfordern zusätzlich einen wirtschaftlich nicht vertretbaren Zeitaufwand zum Auftragen des Überzuges. Uberraschend kann bei den erfindungsgemäßen Pellets durch Auswahl der geeigneten Filmzusatzstoffe die Filmauftragsdicke relativ gering gehalten werden.

Die schnellzerfallenden Pellets können weiterhin mit leichtlöslichen Filmen (z.B. Hydroxypropylmethylcellulose) oder mit Filmen uberzogen werden, die sich im Magen-Darm- Trakt pH- abhängig auflösen (magensaftresistente Überzuge). Die Pellets können auch schichtenweise mit verschiedenen Filmbildnern oder verschiedenen Rezepturen des gleichen Filmbildners überzogen werden. Überzogene Pellets, die nach verschiedenen Verzögerungszeiten den Wirkstoff freigeben, sowie schnellzerfallende nicht überzogene Pellets können gemischt werden, um diverse Freisetzungsprofile (z. B. gepulste Freisetzung, Freigabe nach einer Kinetik 0. Ordnung oder n -ter Ordnung, sigmoidale Freisetzung) einstellen zu können. Die schnellzerfallenden Pellets konnen nach pharmazeutisch gebräuchlichen Methoden überzogen werden, z.B in der Wirbelschicht oder im Dragierkessel.

Vom Zeitpunkt des Freisetzungsbeginns gerechnet weisen die erfindungsgemäßen uberzogenen schnellzerfallenden Pellets eine Freisetzung von mindestens 50% nach 180 Minuten auf. Die Auftragsmenge des Überzuges kann dahingehend variiert werden, daß der Gewichtsanteil des Filmbildners zwischen 1 und 70% bezogen auf das Pelletkerngewicht beträgt.

Als Uberzugsmaterialien für die Herstellung gecoateter Pellets, deren Film durch die Quellung des Pelletkernes gesprengt wird, kommen bevorzugt Ethylcellulose, z.B. Aquacoat® und Methacryiester-Copolymere, z.B. Eudragit® RL/ RS in Frage. Als Materialien für leichtlösliche Filme können z.B. Cellulosederivate (wie z.B. Hydroxypropylmethylcellulose) oder Amino-alkylmethacrylat- Copolymere (z.B.Eudragit® E) eingesetzt werden. Als Filmbildner für pH-abhängig lösliche Überzuge eignen sich beispielsweise Dicarbonsäurederivate von Celluloseverbindungen (z.B.:Hydroypropylmethylcellulosephthalat, Hydroxypropylmethylcellulosesuccinat) sowie Methacrylsäure-Copolymere (z.B. Eudragit® L, Eudragit® S).

Als Filmzusatzstoffe können eingesetzt werden: Weichmacher in einer Menge von 0,1 - 50 %, Antiklebemittel (0,1 - 70 %) sowie, insbesondere bei Filmen, die durch Quellung des Pelletkernes gesprengt werden, Stoffe, die die Diffusion der Freisetzungsflüssigkeit in den Kern beschleunigen oder verzogern können und dadurch die Verzogerungszeit bis zum Start der Wirkstofffreisetzung modifizieren können (0,1 - 50 %). Weiterhin können Porenbildner, Aromastoffe, Farbstoffe, Pigmente sowie Fullstoffe zugesetzt werden.

Als Weichmacher können alle pharmazeutisch üblichen Weichmacher eingesetzt werden. Bevorzugt werden Acetylierte Fettsäureglyceride, Acetyltriethylcitrat, Acetyltributylcitrat, Dibutylphthalat, Diethylphthalat, Dimethylphthalat, Glyceroltriacetat, Propylenglycol, Polyethylenglycol, Polyoxyethylen-Polyoxypropylen-Copolymere, Rizinusöl und Tributylcitrat. Besonders bevorzugt ist Triethylcitrat.

Als Antiklebemittel können alle pharmazeutisch gebräuchlichen Antiklebemittel eingesetzt werden. Bevorzugt werden Talkum, Aerosil®, Kaolin, mikronisierte Kieselsäure. Besonders bevorzugt werden Glycerinester- und ether höherer Fettsäuren, z.B. Glycerinmonostearat, Polyethylenglycol-32- glyceryl- laurat.

Als Zusatzstoffe, die die Diffusion des Wassers in den Pelletkern steuern können, kommen hydrophobierende Zusätze (z. B. Wachse, Talkum, Fettsauren und Fettsäureester) sowie diffusionsbeschleunigende Stoffe (z. B. Tenside, Fettsäureester und Fettsäureether) in Frage, Besonders bevorzugt sind Montanglycolwachs, Glycerinmonostearat, Stearinsäure und Stearinsäurederivate bzw. Polyethylenglycol-glycerylester, Glycerylbehenat, Glycerylpalmitostearat, Cetylpalmitat.

Nach an sich bekannten Verfahren können die erfindungsgemaßen überzogenen und/oder nicht überzogenen Pellets auch mit pharmazeutisch ublichen Hilfsstoffen zu Tabletten verpreßt bzw in Kapseln oder Sachets abgefüllt oder in Matrices eingebettet werden. Die Formulierungen sind gleichermaßen für leicht und schwer wasserlösliche Wirkstoffe geeignet. Die Kapseln enthalten den Wirkstoff in einer Menge bis zu 350 mg, insbesondere 1 - 200 mg, bevorzugt 10 - 100 mg. Pellettabletten konnen den Wirkstoff in einer Menge bis zu 1000 mg, vorzugsweise 1 - 500 mg, insbesondere 10 - 250 mg enthalten.

Anschließend soll die Erfindung an Ausführungsbeispielen näher erläutert werden.

### Beispiel 1

1250 g Carvedilol, 1682,5 g Lactose, 1150 g mikrokristalline Cellulose, 172,5 g Natrium-Dodecylsulfat, 345 g Povidon K 25 und 1150 g Primojel werden in einem Intensivmischer homogenisiert. Die Pulvermischung wird mit ca. 3500 ml destilliertem Wasser granuliert. Anschließend wird die feuchte Masse in einem (Doppelschnecken-) Extruder bei Raumtemperatur extrudiert. Das Extrudat wird dann in einem Runder zu Pellets geformt. Nach dem Trocknen in der Wirbelschicht werden die Pellets gesiebt (Maschenweite 0,6- 1,25 mm).

### Beispiel 2

Analog Beispiel 1 werden Pellets folgender Rezeptur hergestellt:

| | Gewichtsteile |
|---|---|
| Carvedilol | 21,75 |
| Hydroxypropylmethylcellulose 2910 | 3,00 |
| Lactose | 29,25 |
| Mikrokristalline Cellulose | 20,00 |
| Natrium-Dodecylsulfat | 3,00 |
| Povidon K 25 | 3,00 |
| Primojel | 20,00 |
| destilliertes Wasser | 66,70 |

### Beispiel 3

Analog Beispiel 1 werden Pellets folgender Rezeptur hergestellt:

| | Gewichtsteile |
|---|---|
| Carvediloi Carvedilol | 21,75 |
| Hydroxypropylmethylcellulose 2910 | 3,00 |
| Lactose | 37,25 |
| Mikrokristalline Cellulose | 20,00 |
| Natrium-Dodecylsulfat | 3,00 |
| Primojel | 15,00 |
| destilliertes Wasser | 53,85 |

### Beispiel 4

Analog Beispiel 1 werden Pellets folgender Rezeptur hergestellt:

| | Gewichtsteile |
|---|---|
| Carvedilol | 21,75 |
| Lactose | 39,25 |
| Mikrokristalline Cellulose | 10,00 |
| Natrium- Dodecylsulfat | 3,00 |
| Povidon K 25 | 6,00 |
| Primojel | 20,00 |
| destilliertes Wasser | 45,00 |

### Beispiel 5

Analog Beispiel 1 werden Pellets folgender Rezeptur hergestellt:

| | Gewichtsteile |
|---|---|
| Carvedilol | 21,75 |
| Lactose | 9,25 |
| Mikrokristalline Cellulose | 40,00 |
| Natrium- Dodecylsulfat | 3,00 |
| Povidon K 25 | 6,00 |
| Primojel | 20,00 |
| destilliertes Wasser | 81,80 |

### Beispiel 6

Analog Beispiel 1 werden Pellets folgender Rezeptur hergestellt:

| | Gewichtsanteile |
|---|---|
| Carvedilol | 21,75 |
| Lactose | 49,25 |
| Mikrokristalline Cellulose | 10,00 |
| Natrium- Dodecylsulfat | 3,00 |
| Povidon K 25 | 6,00 |
| Primojel | 10,00 |
| destilliertes Wasser | 43.00 |

### Beispiel 7

Analog Beispiel 1 werden Pellets folgender Rezeptur hergestellt:

| | Gewichtsanteile |
|---|---|
| Carvedilol | 21,75 |
| Lactose | 19,25 |
| Mikrokristalline Cellulose | 20,00 |
| Natrium- Dodecylsulfat | 3,00 |
| Povidon K 25 | 6,00 |
| Primojel | 30,00 |
| destilliertes Wasser | 66,70 |

### Beispiel 8

Analog Beispiel 1 werden Pellets folgender Rezeptur hergestellt:

| | Gewichtsanteile |
|---|---|
| Carvedilol | 21,75 |
| Lactose | 29,25 |
| Mikrokristalline Cellulose | 20,00 |
| Natrium- Dodecylsulfat | 3,00 |
| Povidon K 25 | 6,00 |
| Starch 1500 | 20,00 |
| destilliertes Wasser | 33.30 |

### Beispiel 9

Analog Beispiel 1 werden Pellets folgender Rezeptur hergestellt:

| | Gewichtsanteile |
|---|---|
| Carvedilol | 43,50 |
| Lactose | 7,5 |
| Mikrokristalline Cellulose | 20,00 |
| Natrium- Dodecylsulfat | 3,00 |
| Povidon K 25 | 6,00 |
| Primojel | 20,00 |
| destilliertes Wasser | 58.70 |

### Beispiel 10

Analog Beispiel 1 werden Pellets folgender Rezeptur hergestellt:

| | Gewichtsanteile |
|---|---|
| Carvedilol | 21,75 |
| Hydroxypropylmethylcellulose 2910 | 3,00 |
| Lactose | 30,25 |
| Mikrokristalline Cellulose | 20,00 |
| Natrium- Dodecylsulfat | 5,00 |
| Primojel | 20,00 |
| destilliertes Wasser | 69,50 |

### Beispiel 11

| | Gewichtsanteile |
|---|---|
| Carvedilol | 21,75 |
| Lactose | 31,75 |
| Mikrokristalline Cellulose | 20,00 |
| Pluronic F 68 | 0,50 |
| Povidon K 25 | 6,00 |
| Primojel | 20,00 |
| destilliertes Wasser | 43,00 |

Carvedilol, Lactose, Mikrokristalline Cellulose, Primojel und Povidon K 25 werden gemischt. Pluronic F 68 wird in destilliertem Wasser gelöst. Die Pulvermischung wird mit dieser Lösung granuliert. Anschließend wird analog Beispiel 1 extrudiert, gerundet, getrocknet und gesiebt.

### Beispiel 12

| | Gewichtsanteile |
|---|---|
| Carvedilol | 21,75 |
| Lactose | 31,25 |
| Mikrokristalline Cellulose | 20,00 |
| Pluronic F 68 | 1,00 |
| Povidon K 25 | 6,00 |
| Primojel | 20,00 |
| destilliertes Wasser | 43,00 |

Carvedilol, Lactose, Mikrokristalline Cellulose, Primojel und Povidon K 25 werden gemischt. Pluronic F 68 wird in destilliertem Wasser gelöst. Die Pulvermischung wird mit dieser Lösung granuliert. Anschließend wird analog Beispiel 1 extrudiert, gerundet, getrocknet und gesiebt.

### Beispiel 13

| | Gewichtsanteile |
|---|---|
| Carvedilol | 21,75 |
| Lactose | 30,25 |
| Mikrokristalline Cellulose | 20,00 |
| Pluronic F 68 | 2,00 |
| Povidon K 25 | 6,00 |
| Primojel | 20,00 |
| destilliertes Wasser | 43,00 |

Carvedilol, Lactose, Mikrokristalline Cellulose, Primojel und Povidon K 25 werden gemischt. Pluronic F 68 wird in destilliertem Wasser gelöst. Die Pulvermischung wird mit dieser Lösung granuliert. Anschließend wird analog Beispiel 1 extrudiert, gerundet, getrocknet und gesiebt.

### Beispiel 14

| | Gewichtsanteile |
|---|---|
| Carvedilol | 21,75 |
| Lactose | 29,25 |
| Mikrokristalline Cellulose | 20,00 |
| Pluronic F 68 | 3,00 |
| Povidon K 25 | 6,00 |
| Primojel | 20,00 |
| destilliertes Wasser | 64,00 |

Carvedilol, Lactose, Mikrokristalline Cellulose, Primojel und Povidon K 25 werden gemischt. Pluronic F 68 wird in destilliertem Wasser gelöst. Die Pulvermischung wird mit dieser Lösung granuliert. Anschließend wird analog Beispiel 1 extrudiert, gerundet, getrocknet und gesiebt.

### Beispiel 15

| | Gewichtsanteile |
|---|---|
| Carvedilol | 21,75 |
| Lactose | 27,25 |
| Mikrokristalline Cellulose | 20,00 |
| Pluronic F 68 | 5,00 |
| Povidon K 25 | 6,00 |
| Primojel | 20,00 |
| destilliertes Wasser | 33,00 |

Carvedilol, Lactose, Mikrokristalline Cellulose, Primojel und Povidon K 25 werden gemischt. Pluronic® F 68 wird in destilliertem Wasser gelöst. Die Pulvermischung wird mit dieser Lösung granuliert. Anschließend wird analog Beispiel 1 extrudiert, gerundet, getrocknet und gesiebt.

### Beispiel 16

| | Gewichtsanteile |
|---|---|
| Carvedilol | 21,75 |
| Lactose | 29,25 |
| Mikrokristalline Cellulose | 20,00 |
| Mirj | 3,00 |
| Povidon K 25 | 6,00 |
| Primojel | 20,00 |
| destilliertes Wasser | 61,00 |

Carvedilol, Lactose, Mikrokristalline Cellulose, Primojel und Povidon 25 werden gemischt. Mirj wird in destilliertem Wasser gelöst. Die Pulvermischung wird mit dieser Lösung granuliert. Anschließend wird analog Beispiel 1 extrudiert, gerundet, getrocknet und gesiebt.

### Beispiel 17

Analog Beispiel 1 werden Pellets folgender Rezeptur hergestellt:

| | Gewichtsanteile [%] |
|---|---|
| Carvedilol | 21,75 |
| Lactose | 26,25 |
| Mikrokristalline Cellulose | 20,0 |
| Natriumdodecylsulfat | 3,0 |
| Polyethylenglycol-32-glyceryl-laurat | 3,0 |
| Povidon K 25 | 6,0 |
| Primojel | 20,0 |
| destilliertes Wasser | 43,0 |

Carvedilol, Lactose, Mikrokristalline Cellulose, Natriumdodecylsulfat, Povidon K 24 und Priimojel werden gemischt Polyethylenglycol-32-glyceryllaurat wird in destilliertem Wasser geldst. Die Pulvermischung wird mit dieser Losung granuliert. Anschließend wird analog Bsp. 1 extrudiert, gerundet, getrocknet und gesiebt.

### Beispiel 18

Analog Beispiel 1 werden Pellets folgender Rezeptur hergestellt:

| | Gewichtsanteile |
|---|---|
| Carvedilol | 21,75 |
| Glucose | 29,25 |
| Mikrokristalline Cellulose | 20,00 |
| Natrium-Dodecylsulfat | 3,00 |
| Povidon K 25 | 6,00 |
| Primojel | 20,00 |
| destilliertes Wasser | 42,85 |

### Beispiel 19

Analog Beispiel 1 werden Pellets folgender Rezeptur hergestellt:

| | Gewichtsanteile |
|---|---|
| Carvedilol | 21,75 |
| Hydroxypropylmethylcellulose 2910 | 3,00 |
| Mikrokristalline Cellulose | 20,00 |
| Natrium-Dodecylsulfat | 3,00 |
| Primojel | 15,00 |
| Saccharose | 37,25 |
| destilliertes Wasser | 42,85 |

### Beispiel 20

Analog Beispiel 1 werden Pellets folgender Rezeptur hergestellt:

| | Gewichtsanteile |
|---|---|
| Lactose | 71,00 |
| Mikrokristalline Cellulose | 10,00 |
| Natrium-Dodecylsulfat | 3,00 |
| Povidon K 25 | 6,00 |
| Primojel | 10,00 |
| destilliertes Wasser | 20,50 |

### Beispiel 21

Analog Beispiel 1 werden Pellets folgender Rezeptur hergestellt:

| | Gewichtsanteile |
|---|---|
| Glibenclamid | 21,75 |
| Lactose | 29,25 |
| Mikrokristalline Cellulose | 20,00 |
| Natrium-Dodecylsulfat | 3,00 |
| Povidon K 25 | 6,00 |
| Primojel | 20,00 |
| destilliertes Wasser | 25,00 |

### Beispiel 22

Analog Beispiel 1 werden Pellets folgender Rezeptur hergestellt:

| | Gewichtsanteile |
|---|---|
| Carvedilol | 21,75 |
| Lactose | 32,25 |
| Mikrokristalline Cellulose | 20,00 |
| Povidon K 25 | 6,00 |
| Primojel | 20,00 |
| destilliertes Wasser | 72,40 |

### Beispiel 23

Zum Vergleich wurde nur ein Sprengmittel (kein Tensid) eingesetzt, das jedoch allein keinen Zerfall der Pellets ermöglichte. Analog Beispiel 1 werden Pellets folgender Rezeptur hergestellt:

| | Gewichtsanteile |
|---|---|
| Carvedilol | 21,75 |
| Hydroxypropylmethylcellulose 2910 | 3,00 |
| Lactose | 40,25 |
| Mikrokristalline Cellulose | 20,00 |
| Primojel | 15,00 |
| destilliertes Wasser | 53,85 |

**Tabelle 1**

| Beispiel | Aussehen | Zerfall [min] | Freisetzung > 90% [min] |
|---|---|---|---|
| 1 | ++ | <5 | 10-15 |
| 2 | ++ | <5 | 15-20 |
| 3 | ++ | <5 | 20 |
| 4 | ++ | <5 | 12 |
| 5 | ++ | <5 | 20 |
| 6 | + | <3 | n b |
| 7 | - | 10 | 30-40 |
| 8 | ++ | <5 | n.b. |
| 9 | ++ | <3 | 25 |
| 10 | ++ | 3 | 25 |
| 11 | ++ | 2 | n.b. |
| 12 | ++ | <2 | n.b. |
| 12 | ++ | <2 | n.b. |
| 14 | ++ | <2 | 5 |
| 15 | ++ | <2 | n.b. |
| 16 | ++ | <3 | 10 |
| 17 | ++ | <3 | 5-10 |
| 18 | 0 | <5 | n.b. |
| 19 | ++ | <10 | 30 |
| 20 | + | <5 | n.b. |
| 21 | + | <5 | n.b. |
| 22 | ++ | 4 | 20 |
| 23 | ++ | kein Zerfall | 120 |
| Legende zu Tabelle 1: ++ rund | | | |
| + rund- oval | | | |
| 0 oval - oval-stäbchenformig | | | |
| -- Stäbchen | | | |
| n.b. nicht bestimmt | | | |

### Beispiel 24

Bei folgender Zusammensetzung der Rezeptur erfolgt ein Zerfall der Pellets innerhalb von weniger als 3 Minuten:

| Bestandteile der Rezeptur | Gewichtsanteil | Zerfall |
|---|---|---|
| Carvedilol | 70% | < 3 Minuten |
| Lactose D200 | 1% | |
| Mikrokristalline Cellulose | 10% | |
| PVP K 25 | 6% | |
| Natrium-Carboxymethylstarke | 10% | |
| Natrium-Dodecylsulfat | 3% | |
| Wasser | 39% | |

### Beispiel 25

Bei folgender Zusammensetzung der Rezeptur, bei der der Gehalt an Carvedilol unterhalb 25 % liegt, ist kein Zerfall der Pellets festzustellen.

| | | |
|---|---|---|
| Carvedilol | 21,75% | kein Zerfall |
| Lactose D 200 | 49,25% | |
| Mikrokristalline Cellulose | 20% | |
| PVP K 25 | 6% | |
| Natrium-Dodecylsulfat | 3% | |
| Wasser | 22% | |

### Beispiel 26

Analog zu Beispiel 24 wird folgende Rezeptur hergestellt, wobei der Anteil an Natrium-Dodecylsulfat entfällt und der Anteil an Lactose entsprechend erhoht wird. Es ist kein Zerfall der Pellets festzustellen.

| | | |
|---|---|---|
| Carvedilol | 21,75% | kein Zerfall |
| Lactose | 52,25% | |
| Mikrokrisalline Cellulose | 20% | |
| PVP K 25 | 6% | |
| Wasser | 22% | |

### Beispiel 27

Schnellzerfallende Pellets gemäß Bsp. 1 werden in der Wirbelschicht (Hüttlin Kugelcoater) mit einem Film der folgenden Zusammensetzung überzogen:

| Bestandteile der Rezeptur | Gewichtsanteile bezogen auf das Pelletkerngewicht [%] |
|---|---|
| Ethylcellulose (Aquacoat®) | 15% |
| Triethylcitrat | 3% |
| Glycerinmonostearat | 0,75% |
| Destilliertes Wasser | q.s. |

### Beispiel 28

Schnellzerfallende Pellets gemäß Bsp. 1 werden in der Wirbelschicht (Hüttlin Kugelcoater) mit einem Film der folgenden Zusammensetzung uberzogen:

| Bestandteile der Rezeptur | Gewichtsanteile bezogen auf das Pelletkerngewicht |
|---|---|
| Ethylcellulose (Aquacoat®) | 20% |
| Triethylcitrat | 4% |
| Glycerinmonostearat | 1% |
| Destilliertes Wasser | q.s |

### Beispiel 29

Schnellzerfallende Pellets gemäß Bsp. 1 werden in der Wirbelschicht (Hüttlin Kugelcoater) mit einem Film der folgenden Zusammensetzung überzogen:

| Bestandteile der Rezeptur | Gewichtsanteile bezogen auf das Pelletkerngewicht |
|---|---|
| Ethylcellulose (Aquacoat®) | 25% |
| Triethylcitrat | 5% |
| Glycerinmonostearat | 1.25% |
| Destilliertes Wasser | q.s. |

### Beispiel 30

Schnellzerfallende Pellets gemäß Bsp. 1 werden in der Wirbelschicht (Hüttlin Kugelcoater) mit einem Film der folgenden Zusammensetzung überzogen

| Bestandteile der Rezeptur | Gewichtsanteile bezogen auf das Pelletkerngewicht |
|---|---|
| Ethylcellulose (Aquacoat®) | 20% |
| Triethylcitrat | 4% |
| Stearinsaure | 2% |
| Destilliertes Wasser | q.s. |

### Beispiel 31

Schnellzerfallende Pellets gemäß Bsp. 1 werden in der Wirbelschicht (Hüttlin Kugelcoater) mit einem Film der folgenden Zusammensetzung uberzogen:

| Bestandteile der Rezeptur | Gewichtsanteile bezogen auf das Pelletkerngewicht |
|---|---|
| Ethylcellulose (Aquacoat®) | 20% |
| Triethylcitrat | 4% |
| PEG-32-glyceryl-laurat | 2% |
| Destilliertes Wasser | q.s. |

### Beispiel 32

Schnellzerfallende Pellets gemäß Bsp. 1 werden in der Wirbelschicht (Huttlin Kugelcoater) mit einem Film der folgenden Zusammensetzung uberzogen:

| Bestandteile der Rezeptur | Gewichtsanteile bezogen auf das Pelletkernsewicht |
|---|---|
| Ethylcellulose (Aquacoat®) | 20% |
| Triethylcitrat | 4% |
| Montanglycolwachs | 3% |
| Destilliertes Wasser | q.s. |

### Beispiel 33

Schnellzerfallende Pellets gemäß Bsp. 1 werden in der Wirbelschicht (Hüttlin Kugelcoater) mit einem Film der folgenden Zusammensetzung überzogen.

| Bestandteile der Rezeptur | Gewichtsanteile bezogen auf das Pelletkerngewicht |
|---|---|
| Ethvlcellulose (Aquacoat®) | 20% |
| Triethylcitrat | 4% |
| Talkum | 10% |
| Destilliertes Wasser | q.s. |

### Beispiel 34

Schnellzerfallende Pellets gemäß Bsp. 23 werden in der Wirbelschicht (Hüttlin Kugelcoater) mit einem Film der folgenden Zusammensetzung überzogen:

| Bestandteile der Rezeptur | Gewichtsanteile bezogen auf das Pelletkerngewicht |
|---|---|
| Ethylcellulose (Aquacoat®) | 20% |
| Triethylcitrat | 4% |
| Montanglycolwachs | 3% |
| Destilliertes Wasser | q.s. |

### Beispiel 35

Schnellzerfallende Pellets gemaß Bsp. 1 werden in der Wirbelschicht (Hüttlin Kugelcoater) zunächst mit einem Film der folgenden Zusammensetzung überzogen:

| Bestandteile der Rezeptur | Gewichtsanteile bezogen auf das Pelletkerngewicht |
|---|---|
| PEG-32-glyceryl-laurat | 2,5% |
| Destilliertes Wasser | q.s |

Anschließend wird ein Film folgender Zusammensetung aufgetragen:

| Bestandteile der Rezeptur | Gewichtsanteile bezogen auf das Pelletkerngewicht |
|---|---|
| Ethylcellulose (Aquacoat®) | 20% |
| Triethylcitrat | 4% |
| Montanglycolwachs | 3% |
| Destilliertes Wasser | q.s. |

### Beispiel 36

Schnellzerfallende Pellets gemäß Bsp. 1 werden in der Wirbelschicht (Hüttlin Kugelcoater) mit einem Film der folgenden Zusammensetzung uberzogen

| Bestandteile der Rezeptur | Gewichtsanteile bezogen auf das Pelletkerngewicht |
|---|---|
| Ethylcellulose (Aquacoat®) | 20% |
| Triethylcitrat | 4% |
| Montanglycolwachs | 3% |
| PEG-32-glyceryl-laurat | 2% |
| Destilliertes Wasser | q.s. |

### Beispiel 37

Schnellzerfallende Pellets gemäß Bsp. 1 werden in der Wirbelschicht (Hüttlin Kugelcoater) mit einem Film der folgenden Zusammensetzung überzogen:

| Bestandteile der Rezeptur | Gewichtsanteile bezogen auf das Pelletkerngewicht |
|---|---|
| Eudragit RS | 20% |
| Triethylcitrat | 4% |
| Glycerinmonostearat | 0,75% |
| Destilliertes Wasser | q.s. |

### Beispiel 38

Schnellzerfallende Pellets gemäß Bsp. 1 werden in der Wirbelschicht (Hüttlin Kugelcoater) mit einem Film der folgenden Zusammensetzung überzogen:

| Bestandteile der Rezeptur | Gewichtsanteile bezogen auf das Pelletkerngewicht |
|---|---|
| Hydroxypropylmethylcellulose 2910 | 5% |
| Glycerinmonostearat | 0,5% |
| Macrogol 6000 | 1% |
| Destilliertes Wasser | q.s. |

### Beispiel 39

Schnellzerfallende Pellets gemäß Bsp. 1 werden in der Wirbelschicht (Hüttlin Kugelcoater) mit einem Film der folgenden Zusammensetzung überzogen:

| Bestandteile der Rezeptur | Gewichtsanteile bezogen auf das Pelletkerngewicht |
|---|---|
| Eudragit L30 D | 20% |
| Triethylcitrat | 4% |
| Glycerinmonostearat | 1% |
| Polysorbat 80 | 0,02 |
| Destilliertes Wasser | q.s. |

**Tabelle 2**

| Beispiel | Verzögerungszeit [Min] | Freisetzung >50% [Min] (gerechnet ab Beginn der Wirkstofffreisetzung) |
|---|---|---|
| 27 | 40 | 50 |
| 28 | 120 | 120 |
| 29 | 180 | 150 |
| 30 | 180 | 120 |
| 31 | 60 | 40 |
| 32 | 180 | 100 |
| 33 | 100 | 80 |
| 34 | 50 | 30 |
| 35 | 15 | 5 |
| 36 | 90 | 45 |
| 37 | 40 | 120 |
| 38 | 5 | 10 |
| 39 | >120 Min in künstlichem Magensaft | 10 Min in künstlichem Darmsaft |

## Patentansprüche

1. Pharmazeutische Darreichungsformen in Form von Pellets, enthaltend einen oder mehrere Wirkstoffe und mindestens ein als Retardierungs- oder Zerfaltsverzögerungsmittel wirkendes, zur Herstellung von Extrusionspellets erforderliches Rundungsmittel, ein Tablettensprengmittel und mindestens einen Hilfsstoff ausgewählt aus der Gruppe bestehend aus Tensiden und Bindemitteln oder Kombinationen dieser Hilfsstoffe, wobei das Bindemittel Polyvinylpyrrolidon ist.

2. Pharmazeutische Darreichungsform nach Anspruch 1, **dadurch gekennzeichnet, dass** das enthaltene Rundungsmittel mikrokristalline Cellulose ist.

3. Pharmazeutische Darreichungsform nach einem der Ansprüche 1 bis 2, **dadurch gekennzeichnet, dass** die Pellets ein Tensid enthalten.

4. Pharmazeutische Darreichungsform nach einem der Ansprüche 1 bis 3 umfassend mikrokristalline Cellulose, ein oder mehrere Sprengmittel, ein oder mehrere Tenside und/oder ein oder mehrere Bindemittel, sowie gegebenenfalls ein oder mehrere Füllmittel und gegebenenfalls ein oder mehrere Filmbildner.

5. Pharmazeutische Darreichungsform nach einem der Ansprüche 1 bis 4, **dadurch gekennzeichnet, dass** als Wirkstoffe Carvedilol, Bezafibrat, Glibenclamid oder Torasemid enthalten sind.

6. Pharmazeutische Darreichungsform nach einem der Ansprüche 1 bis 5, **dadurch gekennzeichnet, dass** der Wirkstoffanteil bis zu 80 Gew.-% beträgt.

7. Pharmazeutische Darreichungsform nach einem der Ansprüche 1 bis 6, **dadurch gekennzeichnet, dass** die Zerfallszeit der Pellets nicht mehr als 30 Minuten beträgt.

8. Pharmazeutische Darreichungsform nach einem der Ansprüche 1 bis 7, **dadurch gekennzeichnet, dass** die Pellets mit einem die Wirkstofffreisetzung retardierenden Film überzogen sind.

9. Pharmazeutische Darreichungsform nach Anspruch 8, **dadurch gekennzeichnet, dass** der Beginn der Wirkstofffreisetzung nach 10 Minuten bis 5 Stunden erfolgt.

10. Pharmazeutische Darreichungsform nach einem der Ansprüche 1 bis 9, **dadurch gekennzeichnet, dass** das Sprengmittel Natrium-Carboxymethylcellulose, modifizierte Maisstärke oder Natrium-Carboxymethylstärke ist.

11. Pharmazeutische Darreichungsform nach einem der Ansprüche 1 bis 10, **dadurch gekennzeichnet, dass** das Tensid Benzalkoniumchlorid, Polyoxyethylen-Polyoxypropylen-Copolymere, Polyethylen-glycolglycerylester, NatriumDodecylsulfat oder PEG-400 Stearat ist.

12. Pharmazeutische Darreichungsform nach einem der Ansprüche 1 bis 11, **dadurch gekennzeichnet, dass** das Füllmittel ein Kohlenhydrat, wie Zucker, vorzugsweise Glucose, Lactose und Saccharose; Zuckeralkohol, wie Mannit und Sorbit; Stärke; Stärkederivate oder Dibasisches Calciumphosphat ist.

13. Pharmazeutische Darreichungsform nach einem der Ansprüche 1 bis 12 enthaltend 5-70 Gew.-% mikrokristalline Cellulose, vorzugsweise 15-25 Gew.-%.

14. Pharmazeutische Darreichungsform nach einem der Ansprüche. 1 bis 13 enthaltend 5-50 Gew.-% Tablettensprengmittel, vorzugsweise 15-25 %.

15. Pharmazeutische Darreichungsform nach einem der Ansprüche 1 bis 14 enthaltend 0,1-20 Gew.-% Tenside, vorzugsweise 2-10 %.

16. Pharmazeutische Darreichungsform nach einem der Ansprüche 1 bis 15 enthaltend 1-10 Gew.-% Bindemittel, vorzugsweise 3-7 %.

17. Verfahren zur Herstellung von pharmazeutischen Darreichungsformen nach einem der Ansprüche 1 bis 16, **dadurch gekennzeichnet, dass** man die Wirkstoffe mit den Hilfsstoffen mischt, anschließend granuliert, extrudiert und zu Pellets rundet und danach gegebenenfalls als Tabletten, Kapseln oder Sachets zur Verfügung stellt und/oder gegebenenfalls die Pellets oder die Tabletten mit geeigneten Überzügen (Filmbildnern) versieht.

18. Verfahren nach Anspruch 17, **dadurch gekennzeichnet, dass** die Pellets als Überzugsmaterialien für gecoatete Pellets bevorzugt Ethylcellulose und Methacrylester-Copolymere enthalten.

19. Verfahren nach Anspruch 17 oder 18, **dadurch gekennzeichnet, dass** die Pellets als Überzugsmaterialien für leichtlösliche Filme bevorzugt Cellulosederivate oder Aminoalkylrnethacrylat-Copolymere enthalten.

20. Verfahren nach einem der Ansprüche 17 bis 19, **dadurch gekennzeichnet, dass** die Pellets als Überzugsmaterialien für pH-abhängige lösliche Überzüge Dicarbonsäurederivate von Celluloseverbindungen und Methacrylsäure-Copolymere enthalten.

21. Verfahren nach einem der Ansprüche 17 bis 20, **dadurch gekennzeichnet, dass** die Überzugsmaterialien als Filmzusatzstoffe 0.1-50 % Weichmacher, 0.1-70 % Antiklebemittel und 0.1-50 % Zusatzstoffe zur Steuerung der Diffusion des Wassers in den Pelletkern, enthalten.

22. Verfahren nach einem der Ansprüche 17 bis 21, **dadurch gekennzeichnet, dass** die Pellets als Weichmacher bevorzugt acetylierte Fettsäureglyceride, Acetyltriethylcitrat, Acetyltributylcitrat, Dibutylphthalat, Diethylphthalat, Dimethylphthalat, Glyceroltriacetat, Propylenglycol, Polyethylenglycol; Polyoxyethylen-Polyoxypropylen-Copolymere, Rizinusöl, Triethycitrat oder Tributylcitrat enthalten.

23. Verfahren nach einem der Ansprüche 17 bis 22, **dadurch gekennzeichnet, dass die** Pellets als Weichmacher bevorzugt Triethylcitrat enthalten.

24. Verfahren nach einem der Ansprüche 17 bis 23, **dadurch gekennzeichnet, dass** die Pellets als Anitklebemittel bevorzugt Talkum, Aerosil, mikronisierte Kieselsäure oder Kaolin enthalten.

25. Verfahren nach einem der Ansprüche 17 bis 24, **dadurch gekennzeichnet, dass** die Pellets als Zusatzstoffe, die die Diffusion des Wassers in den Pelletkern steuern können, bevorzugt Montanglycolwachs, Glycerinmonostearat, Stearinsäure und Stearinsäurederivate bzw. Polyethylen-glyceryl-ester, Glycerylbehenat, Glycerylpalmitostearat oder Cetylpalmitat enthalten.

26. Verwendung eines oder mehrerer Tablettensprengmittel in Kombination mit einem oder mehreren Tensiden und/oder einem Bindemittel zur Herstellung von pharmazeutischen Darreichungsformen nach einem der Ansprüche 1 bis 16.

## Claims

1. Pharmaceutical forms of administration in the form of pellets, containing one or more active substances and at least one rounding agent required for the production of extrusion pellets and acting as a retardant or disintegration-retarding agent, a tablet disintegrant and at least one auxiliary substance selected from the group consisting of tensides and binding agents or combinations of these auxiliary substances, the binding agent being polyvinylpyrrolidone.

2. Pharmaceutical form of administration according to claim 1, **characterised in that** the rounding agent contained therein is microcrystalline cellulose.

3. Pharmaceutical form of administration according to one of claims 1 to 2, **characterised in that** the pellets contain a tenside.

4. Pharmaceutical form of administration according to one of claims 1 to 3, comprising microcrystalline cellulose, one or more disintegrants, one or more tensides and/or one or more binding agents, as well as one or more fillers as required and one or more film formers as required.

5. Pharmaceutical form of administration according to one of claims 1 to 4, **characterised in that** carvedilol, bezafibrate, glibenclamide or torasemide are contained therein as active substances.

6. Pharmaceutical form of administration according to one of claims 1 to 5, **characterised in that** the active substance content is up to 80% by weight.

7. Pharmaceutical form of administration according to one of claims 1 to 6, **characterised in that** the disintegration time of the pellets is not more than 30 minutes.

8. Pharmaceutical form of administration according to one of claims 1 to 7, **characterised in that** the pellets are coated with a film which retards the release of the active substance.

9. Pharmaceutical form of administration according to claim 8, **characterised in that** the start of the active substance release takes place after 10 minutes to 5 hours.

10. Pharmaceutical form of administration according to one of claims 1 to 9, **characterised in that** the disintegrant is sodiwn carboxymethyl cellulose, modified com starch or sodium carboxymethyl starch.

11. Pharmaceutical form of administration according to one of claims 1 to 10, **characterised in that** the tenside is benzalkonium chloride, polyoxyethylene polyoxypropylene copolymer, polyethylene glycol glyceryl ester, sodium dodecyl sulphate or PEG-400 stearate.

12. Pharmaceutical form of administration according to one of claims 1 to 11, **characterised in that** the filler is a carbohydrate, such as sugar, preferably glucose, lactose and sucrose; sugar alcohol, such as mannitol and sorbitol; starch; starch derivative or dibasic calcium phosphate.

13. Pharmaceutical form of administration according to one of claims 1 to 12, containing 5-70% by weight microcrystalline cellulose, preferably 15-25% by weight.

14. Pharmaceutical form of administration according to one of claims 1 to 13, containing 5-50% by weight tablet disintegrant, preferably 15-25%.

15. Pharmaceutical form of administration according to one of claims 1 to 14, containing 0.1-20% by weight tenside, preferably 2-10%.

16. Pharmaceutical form of administration according to one of claims 1 to 15, containing 1-10% by weight binding agent, preferably 3-7%.

17. Process for the production of pharmaceutical forms of administration according to one of claims 1 to 16, **characterised in that** the active substances are mixed with the auxiliary substances, subsequently granulated, extruded and rounded into pellets and thereafter made available as required in the form of tablets, capsules or sachets and/or the pellets or the tablets are provided as required with suitable coatings (film formers).

18. Process according to claim 17, **characterised in that** the pellets preferably contain ethyl cellulose and methacrylic ester copolymers as coating materials for coated pellets.

19. Process according to claim 17 or 18, **characterised in that** the pellets preferably contain cellulose derivatives or aminoalkyl methacrylate copolymers as coating materials for readily soluble films.

20. Process according to one of claims 17 to 19, **characterised in that** the pellets contain dicarboxylic acid derivatives of cellulose compounds and methacrylic acid copolymers as coating materials for pH-dependent soluble coatings.

21. Process according to one of claims 17 to 20, **characterised in that** the coating materials contain as film additives 0.1-50% plasticiser, 0.1-70% anti-caking agent and 0.1-50% additives to control the diffusion of water into the pellet core.

22. Process according to one of claims 17 to 21, **characterised in that** the pellets preferably contain acetylated fatty acid glycerides, acetyl triethyl citrate, acetyl tributyl citrate, dibutyl phthalate, diethyl phthalate, dimethyl phthalate, glycerol triacetate, propylene glycol, polyethylene glycol, polyoxyethylene-polyoxypropylene copolymers, castor oil, triethyl citrate or tributyl citrate as plasticiser.

23. Process according to one of claims 17 to 22, **characterised in that** the pellets preferably contain triethyl citrate as plasticiser.

24. Process according to one of claims 17 to 23, **characterised in that** the pellets preferably contain talc, aerosil, micronised silicic acid or kaolin as the anti-caking agent.

25. Process according to one of claims 17 to 24, **characterised in that** the pellets preferably contain montan glycol wax, glycerol monostearate, stearic acid and stearic acid derivatives or, respectively, polyethylene glyceryl ester, glyceryl behenate, glyceryl palmito-stearate or cetyl palmitate as additives which can control the diffusion of water into the pellet core.

26. Use of one or more tablet disintegrants in combination with one or more tensides and/or a binding agent for the production of pharmaceutical forms of administration according to one of claims 1 to 16.

## Revendications

1. Formes pharmaceutiques sous forme de pellets, contenant un ou plusieurs principes actifs et au moins un agent facilitant l'arrondissage, jouant le rôle d'un agent retard ou retardateur de décomposition, nécessaire à la fabrication de pellets par extrusion, un désintégrant pour comprimés et au moins un auxiliaire choisi dans l'ensemble comprenant les tensioactifs et les liants ou des combinaisons de ces auxiliaires, le liant étant de la polyvinylpyrrolidone.

2. Forme pharmaceutique selon la revendication 1, **caractérisée en ce que** l'agent facilitant l'arrondissage qui y est contenu est une cellulose microcristalline.

3. Forme pharmaceutique selon l'une des revendications 1 ou 2, **caractérisée en ce que** les pellets contiennent un tensioactif.

4. Forme pharmaceutique selon l'une des revendications 1 à 3, contenant une cellulose microcristalline, un ou plusieurs désintégrants, un ou plusieurs tensioactifs et/ou un ou plusieurs liants, et éventuellement une ou plusieurs charges et éventuellement un ou plusieurs agents d'enrobage.

5. Forme pharmaceutique selon l'une des revendications 1 à 4, **caractérisée en ce qu'**elle contient en tant que principe actif du carvédilol, du bézafibrate, du glibenclamide ou du torasémide.

6. Forme pharmaceutique selon l'une des revendications 1 à 5, **caractérisée en ce que** la proportion du principe actif va jusqu'à 80 % en poids.

7. Forme pharmaceutique selon l'une des revendications 1 à 6, **caractérisée en ce que** le temps de décomposition des pellets n'est pas supérieur à 30 minutes.

8. Forme pharmaceutique selon l'une des revendications 1 à 7, **caractérisée en ce que** les pellets sont enrobés d'un film retardateur de libération du principe actif.

9. Forme pharmaceutique selon la revendication 8, **caractérisée en ce que** le début de la libération du principe actif a lieu au bout de 10 minutes à 5 heures.

10. Forme pharmaceutique selon l'une des revendications 1 à 9, **caractérisée en ce que** le désintégrant est la carboxyméthylcellulose sodique, l'amidon de maïs modifié ou le carboxyméthylamidon sodique.

11. Forme pharmaceutique selon l'une des revendications 1 à 10, **caractérisée en ce que** le tensioactif est le chlorure de benzalkonium, un copolymère polyoxyéthylènepolyoxypropylène, un ester glycérylique du polyéthylèneglycol, le dodécylsulfate de sodium ou le stéarate de PEG-400.

12. Forme pharmaceutique selon l'une des revendications 1 à 11, **caractérisée en ce que** la charge est un hydrate de carbone tel que le sucre, de préférence le glucose, le lactose ou le saccharose ; un alcool de sucre tel que le mannitol et le sorbitol ; l'amidon ; les dérivés de l'amidon ou le phosphate de calcium dibasique.

13. Forme pharmaceutique selon l'une des revendications 1 à 12, qui contient de 5 à 70 et de préférence de 15 à 25 % en poids de cellulose microcristalline.

14. Forme pharmaceutique selon l'une des revendications 1 à 13, qui contient de 5 à 50 et de préférence de 15 à 25 % en poids d'un désintégrant pour comprimés.

15. Forme pharmaceutique selon l'une des revendications 1 à 14, qui contient de 0,1 à 20 et de préférence de 2 à 10 % de tensioactifs.

16. Forme pharmaceutique selon l'une des revendications 1 à 15, qui contient de 1 à 10 et de préférence de 3 à 7 % en poids de liants.

17. Procédé de fabrication de formes pharmaceutiques selon l'une des revendications 1 à 16, **caractérisé en ce qu'**on mélange les principes actifs aux auxiliaires, puis on granule, on extrude et on arrondit pour obtenir des pellets, puis on les met éventuellement à disposition sous forme de comprimés, de gélules ou de sachets, et/ou on pourvoit éventuellement les pellets ou les comprimés de revêtements appropriés (agents d'enrobage).

18. Procédé selon la revendication 17, **caractérisé en ce que** les pellets contiennent de préférence, en tant que matériaux de revêtement pour pellets enrobés, de l'éthylcellulose ou des copolymères d'esters de l'acide méthacrylique.

19. Procédé selon la revendication 17 ou 18, **caractérisé en ce que** les pellets contiennent de préférence en tant que matériaux de revêtement pour films facilement solubles des dérivés de la cellulose ou des copolymères de méthacrylate d'aminoalkyle.

20. Procédé selon l'une des revendications 17 à 19, **caractérisé en ce que** les pellets contiennent en tant que matériaux de revêtement, pour des enrobages solubles en fonction du pH, des dérivés d'acides dicarboxyliques de composés de la cellulose et des copolymères de l'acide méthacrylique.

21. Procédé selon l'une des revendications 17 à 20, **caractérisé en ce que** les matériaux de revêtement contiennent en tant qu'additifs d'enrobage 0,1 à 50 % de plastifiants, 0,1 à 70 % d'agents anti-adhésifs et 0,1 à 50 % d'additifs destinés à contrôler la diffusion de l'eau dans le coeur du pellet.

22. Procédé selon l'une des revendications 17 à 21, **caractérisé en ce que** les pellets contiennent de préférence, en tant que plastifiants, des glycérides d'acides gras acétylés, de l'acétylcitrate de triéthyle, de l'acétylcitrate de tributyle, du phtalate de dibutyle, du phtalate de diéthyle, du phtalate de diméthyle, du triacétate de glycérol, du propylèneglycol, du polyéthylèneglycol, des copolymères polyoxyéthylène-polyoxypropylène, de l'huile de ricin, du citrate de triéthyle ou du citrate de tributyle.

23. Procédé selon l'une des revendications 17 à 22, **caractérisé en ce que** les pellets contiennent de préférence du citrate de triéthyle en tant que plastifiant.

24. Procédé selon l'une des revendications 17 à 23, **caractérisé en ce que** les pellets contiennent de préférence en tant qu'agent anti-adhésif du talc, de l'Aerosil, de la silice micronisée ou du kaolin.

25. Procédé selon l'une des revendications 17 à 24, **caractérisé en ce que** les pellets contiennent de préférence, en tant qu'additifs qui peuvent contrôler la diffusion de l'eau dans le coeur du pellet, de la cire de lignite, du monostéarate de glycérol, de l'acide stéarique et des dérivés de l'acide stéarique ou des esters glycéryliques du polyéthylène, du béhénate de glycéryle, du palmitostéarate de glycéryle ou du palmitate de cétyle.

26. Utilisation d'un ou plusieurs désintégrants pour comprimés en combinaison avec un ou plusieurs tensioactifs et/ou un liant, pour fabriquer des formes pharmaceutiques selon l'une des revendications 1 à 16.
